# EUROPEAN PATENT SPECIFICATION

(11) **EP 1 690 507 B1**
(45) Date of publication and mention of the grant of the patent: **17.12.2008**
(21) Application number: 03778352.9
(22) Date of filing: 25.11.2003
(51) Int. Cl.: A61B 17/72

(54) **INTRAMEDULLARY NAIL**
MARKNAGEL
CLOU MEDULAIRE

(43) Date of publication of application: 16.08.2006
(73) Proprietor: Instituto Tecnologico de Canarias, S.A. (ITC), 35119 Santa Lucia, Las Palmas (ES); Ara Pinilla, Javier, 35119 Santa Lucia, Las Palmas (ES); De La Barreda Lopez, Guillermo, 35119 Santa Lucia, Las Palmas (ES); Monopoli Forleo, Donato, 35119 Santa Lucia, Las Palmas (ES)
(72) Inventor: ARA PINILLA, Javier, 35119 Santa Lucia, Las Palmas de G.C. (ES); DE LA BARREDA LOPEZ, Guillermo, 35119 Santa Lucia, Las Palmas de G.C. (ES); MONOPOLI FORLEO, Donato, 35119 Santa Lucia, Las Palmas de G.C. (ES)
(74) Representative: PROPI, S.L.
(86) International application number: PCT/ES2003/000597
(87) International publication number: WO 2005/053551

(56) References cited:
- SU-A1- 662 082
- US-A- 3 759 257
- US-A- 4 091 806
- US-A- 4 227 518
- US-A1- 2002 165 544
- US-B1- 6 224 600

## Description

### OBJECT OF THE INVENTION

The present invention relates to an intramedullary nail, of the type used to secure and immobilise fractures in long bones such as the femur.

The object of the invention is to achieve an elastic nail that is easier to implant in the bone, thus causing less damage thereto and improving the fixation, which also helps the bone to knit together.

### BACKGROUND OF THE INVENTION

Nails are usually used to immobilise a long fractured bone, being hammered into one of the ends of the bone and having at each end a pair of holes for receiving respective cross screws that immobilise the nail by securing it to the two parts of the bone and consequently joining said fractured parts to one other.

This solution has certain problems, particularly with regard to the following aspects:
- As the bones to be immobilised are not straight, the nail tends to become deformed as it is hammered in, with the risk of passing through the bone, causing excessive damage to the spongy part thereof, which separates the intramedullary canal from the hard outer layer.
- Its fixation necessitates the use four cross screws and it is quite difficult to line up said screws with the holes in the nail.
- It is necessary to use x-rays or probe holes to locate the holes for the screws, as these are hidden inside the bone.

The difficulty of implanting said cross screws becomes considerably more pronounced in the case of the lower or distal holes, and the use of x-rays is not acceptable to surgeons, as they are frequently exposed to high radiations, particularly on their hands.

Although guiding systems exist in order to help to centre the holes, as do other systems with a "probe halo" wherein a guide is used to indicate the position of the holes in the nail, these solutions do not solve the above-described problems.

A nail called the "Marchetti" nail is known, which attempts to avoid such problems. It consists of a multiple nail, i.e. a plurality of very thin rods, which extend from a common core, so that they "open up" as they are inserted into the intramedullary canal and are driven into the spongy bone tissue, thus ensuring distal fixation without the need for distal nails.

More specifically, this set of thin rods is aided by a ring, which ascends when the nail is half inserted, opening the set of rods so that as they continue to be inserted into the intramedullary canal the thin rods point towards the spongy tissue and are driven into it.

Although this solution substantially simplifies the operating system and minimises the use of x-rays, it does present certain problems with regard to the following aspects:
- It is not very secure.
- The thin filaments or rods can damage the bone.
- The fixation is arbitrary, as the deformation and manner in which the filaments are driven into the bone is not controllable.

In the same place, the document US 2002165544 relates to an intramedullary nail for fixation of a fractured bone having a medullary canal. The intramedullary nail comprising a first radially expandable secction adjent to the head, a second radially expandable adjacent to the tip and a non-expandable section disposed between the first and second radially expandable sections. The head of the intramedullary nail may be provided with a distraction mecanism, by means of which the first and second radially expandable are distractible transverse to said longitudinal axis. The non-espandable section may comprises at least one unslotted tubular piece.

In the present invention, the intramedullary nail is based in a plurality of cylindric rods too, but in the hollow interior of the nail, a probe works with the nail, having a protusion close to its distal end that acts as an expanding element for the terminal section of the rods. Furthermore, there is a support which is solidly fixed to the bone by means of screw and the nail passes therethrogh, immovaby fixing the head, also having a radial fin that has a pair of holes through which the respective locking screws pass.

### DESCRIPTION OF THE INVENTION

The intramedullary nail proposed by the invention successfully solves the above-described problems in each of the aspects that have been mentioned.

More specifically, to do this said nail consists of a functional combination of a nail and a probe that can move axially inside the nail, the purpose this probe being to cause a radial deformation of the nail, so that this need only be fixed by screws at the proximal end of the bone, whilst it is fixed at the distal end by said expansion effect.

This is achieved thanks to the fact that the nail itself has a plurality of filaments extending from a head at its proximal end, which are disposed according to an imaginary cylindrical surface and which converges at a node that is considerably distanced from the head, beyond which said filaments extend in a wide section, the probe including a marked protrusion close to its distal end, so that, once the nail-probe assembly has been implanted inside the bone, as the probe moves upwards said protrusion first causes a radial deformation of the ends of the filaments, which are driven into the spongy bone tissue, and when said protrusion reaches the node of the nail, it in turn causes said node to move towards the head of the nail, which in turn causes the initial section to bulge outwards, thereby adapting and fixing the filaments to the inner wall of the bone.

In order to achieve the aforementioned effect it is necessary for the upward movement of the probe to begin before the nail has fully penetrated the bone, so that after the radial expansion of the free end of the filaments, these are driven into the bone as the nail in turn completes its final forward movement.

In accordance with the description above, it is only necessary to perform one screwing operation, namely the operation of screwing the head of the nail to the proximal end of the bone, which can be done using a complementary template, without the need for x-rays.

### DESCRIPTION OF THE DRAWINGS

To complement this description and in order to aid a better understanding of the invention's characteristics, according to a preferred practical embodiment thereof, there is a set of illustrative and non-limiting drawings integral to said description, which are as follows:
- Figure 1.: Shows a schematic perspective view of an intramedullary nail according to the object of the present invention.
- Figure 2.: Shows a detail of a longitudinal section of the support for the head of the nail.
- Figure 3.: Shows a detail of a perspective view of the nail without its support and without the interior probe.
- Figure 4.: Shows a detail of a perspective view of the tool for fixing said support, in a working position at the corresponding end of the bone.

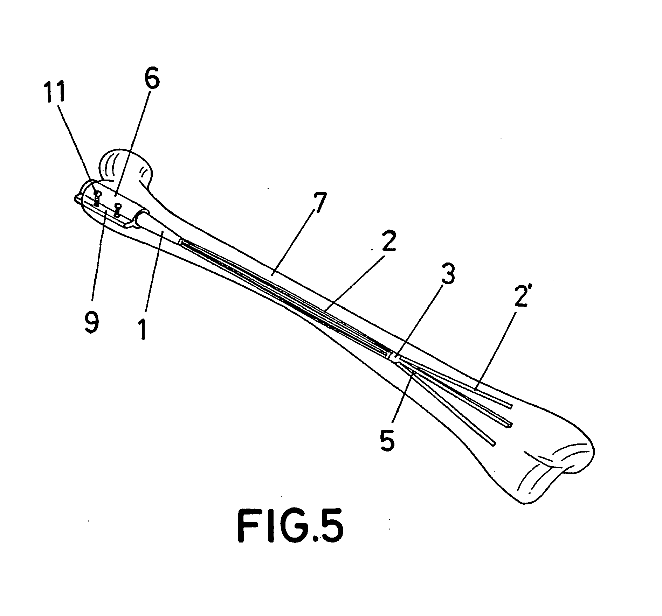

- Figure 5.: Shows another perspective view of the nail assembly, here duly implanted in a femur.

### PREFERRED EMBODIMENT OF THE INVENTION

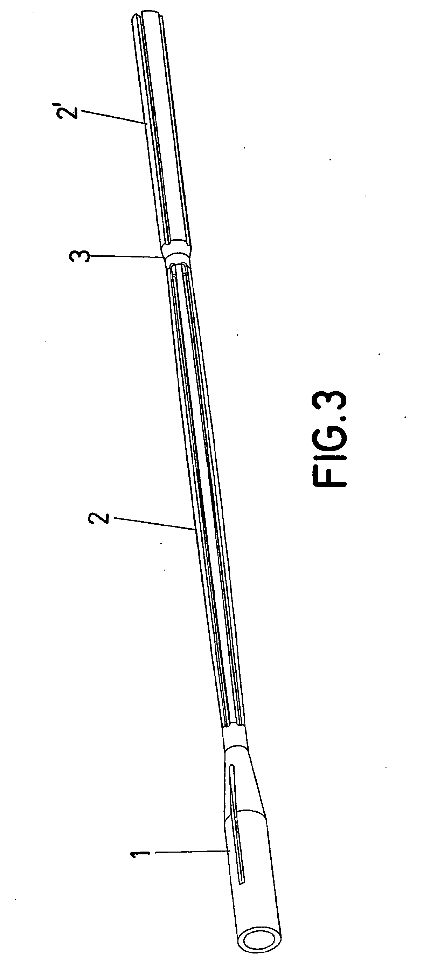

In view of the aforementioned figures, and particularly figure 3, it is possible to observe how the intramedullary nail proposed by the invention consists of a tubular nail formed by a head (1) to which a plurality of rods (2) of a considerable length are joined and from which they extend, being distributed according to an imaginary cylinder of a small diameter and converging at a node (3), beyond which said rods (2) extend in wide sections (2') of considerable length with independent free ends.

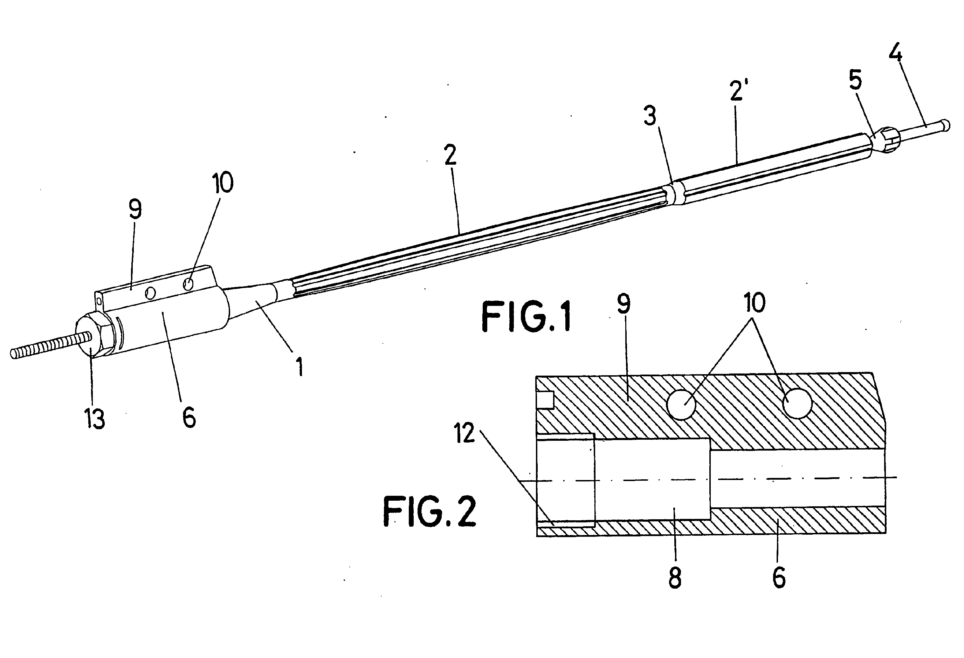

A probe (4) works with tubular the nail (1-2), this probe consisting of a threaded rod that can be housed inside the hollow interior of the tubular nail, having a protrusion (5) close to its distal end that acts as an expanding element for the wide sections (2') of the rods (2), as will be seen below, the threaded rod (4) emerging through the proximal end of the tubular nail, as can be observed in figure 1

A support (6) is solidly fixed to the bone (7) by means of screws and the nail passes therethrough, finally and immovably fixing the head thereof, for which said support (6) has a stepped axial hole (8) to receive said head (1), also having a radial fin that has a pair of holes (10) through which the respective locking screws (11) pass.

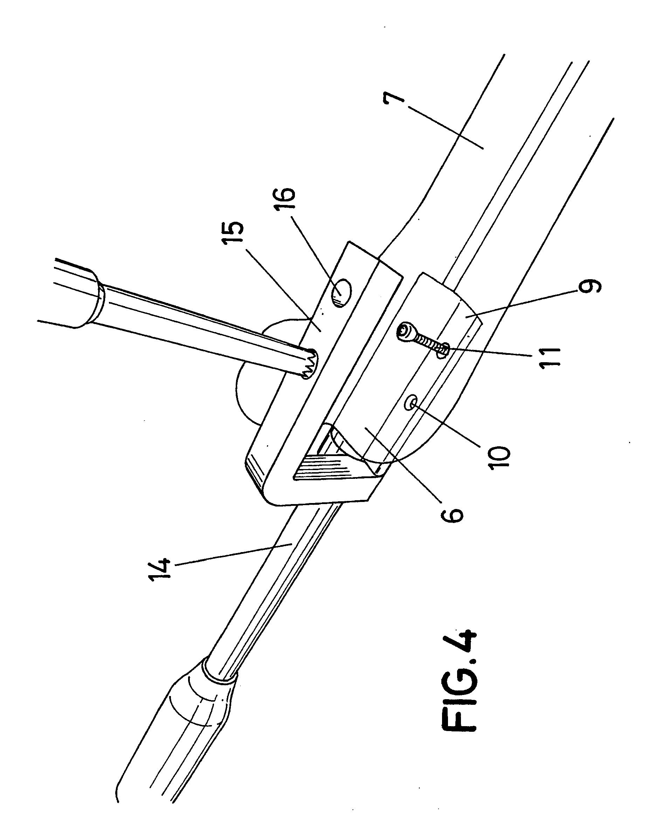

Said hole (8) in the support (6) includes a threaded section (12) at its outer end for the attachment of a collar (13) by means of which the axial traction on the probe (4) is finally performed, and which is initially used for the attachment of a tool (14), shown in figure 4, with a bent arm (15) and a pair of holes (16), so that when said tool (14) is duly attached to the support (6), the holes (16) in said tool are coaxially aligned with the holes (10) in the support (1), thus making it possible to drill holes in the bone (7) with the certainty that the screws (11) will therethrough inevitably reach the holes (10) in the support.

To assemble the intramedullary nail, the support (6) is initially fixed to the proximal end of the fractured bone (7) and is screwed in place, then the assembly consisting of the probe (4) and tubular nail (1-2) is inserted until it reaches a position in which a relative axial movement will occur between the probe (4) and the nail, giving rise to a first phase of divergence of the ends (2') of the rods (2). At that moment, an actuation on the collar (13) causes an axial traction of the probe (4) until it reaches a position in which the protrusion (5) thereon comes into contact with the node (3) of the nail, thereby causing the wide section; (2') of the rods (2) to adopt their maximum divergence and press against the inner wall of the bone.

At this moment, the assembly of the head (1) on the support (6) is completed until it reaches a position in which there will be a longitudinal forward movement of the intramedullary nail, so that the free ends of the rods (2) are driven into the spongy bone tissue, and the proximal section of said rods (2) bulges outwards, i.e. said rods undergo a radial expansion in this area, pressing against the side wall of the bone and thus achieving not only an anti-rotational or anti-torsion tension, but also a longitudinal tension of the bone, which helps it to knit together.

The filaments that are drive into the spongy tissue are controllable and almost reach a perpendicular position relative the bone, which gives the nail greater stability.

The elastic properties of the nail generate longitudinal tension when the patient puts weight on the leg, thus helping the bone to knit together.

The use of x-rays is practically reduced to the follow-up stage, without the need for radiation while the screw is being implanted and, consequently, without said radiation affecting the surgeon's hands.

The special configuration of the intramedullary nail and the manner in which it is assembled enable it to be implanted in the bone by pressure using a suitable nail driver, rather than the classic hammering methods.

## Claims

1. Intramedullary nail, which is specially designed to secure and immobilise fractures in long bones such as the femur, said nail consisting of the functional combination of a tubular nail (1-2-3-2') and a probe (4) that can move axially inside of said tubular nail (1-2-3-2'), which includes a node (3) and a plurality of thin rods (2) of a considerable length extending distally, which are grouped according to an imaginary cylindrical surface and converge towards the node (3), beyond which they extend in considerably wide sections (2') that are independent at their free ends, whilst the probe (4) includes a protrusion (5) close to its distal end, which is initially situated outside the tubular nail, **characterised in that** the tubular nail further includes a head (1) at its proximal end, from which the plurality of thin rods (2) extend distally and the protrusion (5) first causes the radial deformation of the wide sections (2') of the rods (2) during the axial movement of the probe relative to the tubular nail and then causes the node (3) to move towards the head (1), which in turn causes a radial expansion of the tubular nail in the proximal area at its rods (2).

2. An assembly including a support (6) and an intramedullary nail according to claim 1, **characterised in that** the support (6) works with the head (1) of the tubular nail, being the only element of the assembly that is fixed by screws to the bone, specifically at the proximal end thereof, this support (6) having a stepped axial hole (8) for attachment of the head (1) and a radial fin (9) with a pair of holes (10) for screwing the support to the bone.

3. An assembly according to claim 2, **characterised in that** inside the axial hole (8) in the support (6), specifically at the outer end thereof, there is a threaded section (12) for the attachment of a template for drilling into the bone, which is situated in line with the holes (10) of the support (6), and for the subsequent implantation of a collar (13) that can move the threaded rod (4) that constitutes the probe in order to displace the protrusion (5) thereon towards the head (1) of the tubular nail.

## Patentansprüche

1. Marknagel, der insbesondere für die Sicherung und Ruhigstellung von Brüchen von langen Knochen, wie zum Beispiel das Femur, konzipiert ist, wobei der besagte Nagel aus einer funktionalen Kombination aus einem röhrenförmigen Nagel (1-2-3-2') und einer Sonde (4) besteht, welche axial im Innern des besagten röhrenförmigen Nagels (1-2-3-2') bewegt werden kann, welcher einen Knoten (3) und eine Vielzahl von dünnen Stäben (2) mit einer beachtlichen Länge, die sich in distaler Weise erstrecken, einschließt, welche gemäß einer imaginären zylinderförmigen Oberfläche angeordnet sind und welche zu dem Knoten (3) hin konvergieren, über den sie sich in beachtlich weiten Sektionen (2') erstrecken, die an ihren freien Enden unabhängig sind, während die Sonde (4) eine Ausbuchtung (5) einschließt, die nahe an ihrem distalen Ende liegt, die anfangs außerhalb des röhrenförmigen Nagels platziert ist, **dadurch gekennzeichnet, dass** der röhrenförmige Nagel ferner an seinem proximalen Ende einen Kopf (1) einschließt, von dem sich die Vielzahl der dünnen Stäbe (2) distal erstreckt und die Ausbuchtung (5) zunächst die radiale Deformation der weiten Sektionen (2') der Stäbe (2) verursacht, während der axialen Bewegung der Sonde in Bezug zu dem röhrenförmigen Nagel und dann den Knoten (3) dazu bringt, zu dem Kopf (1) bewegt zu werden, welcher wiederum eine radiale Ausdehnung des röhrenförmigen Nagels in dem proximalen Bereich an seinen Stäben (2) verursacht.

2. Eine Anordnung, die eine Schiene (6) und einen Marknagel nach Anspruch 1 einschließt, **dadurch gekennzeichnet, dass** die Schiene (6) mit dem Kopf (1) des röhrenförmigen Nagels funktioniert, wobei es sich um das einzige Element der Anordnung handelt, das durch Schrauben am Knochen befestigt ist, insbesondere an dessen proximalen Ende, wobei diese Schiene (6) über eine gestufte axiale Bohrung (8) verfügt, zur Befestigung des Kopfes (1), und über einen radialer Grat (9) mit einem Paar von Bohrungen (10), um die Schiene an den Knochen zu schrauben.

3. Eine Anordnung nach Anspruch 2, **dadurch gekennzeichnet, dass** sich im Innern der axialen Bohrung (8) in der Schiene (6), insbesondere an deren äußerem Ende, eine Sektion (12) mit Gewinde befindet, zur Befestigung einer Vorlage zum Bohren in den Knochen, welche in einer Linie mit den Bohrungen (10) der Schiene (6) angeordnet ist, und für die anschließende Implantierung eines Bundrings (13), der den Stab (4) mit Gewinde bewegen kann, welcher die Sonde konstituiert, um die Ausbuchtung (5) darauf in Richtung des Kopfes (1) des röhrenförmigen Nagels zu verschieben.

## Revendications

1. Clou intramédullaire, conçu spécialement pour la stabilisation et l'immobilisation des fractures des os longs tels que le fémur, consistant en la combinaison fonctionnelle d'un clou tubulaire (1-2-3-2') et d'une sonde (4) pouvant se déplacer axialement à l'intérieur dudit clou tubulaire (1-2-3-2'), qui comporte un noeud (3) et un ensemble de tiges fines (2) d'une longueur considérable s'étendant distalement, qui sont disposées suivant une surface cylindrique imaginaire et convergent vers le noeud (3), au-delà duquel elles s'étendent en segments de grande taille (2') indépendants à leur extrémité libre, tandis que la sonde (4) comporte une protrusion (5) près de son extrémité distale, qui est initialement située hors du clou tubulaire, **caractérisé en ce que** le clou tubulaire comporte également une tête (1) à son extrémité proximale, depuis laquelle l'ensemble des fines tiges (2) s'étend distalement, la protrusion (5) causant d'abord la déformation radiale des segments de grande taille (2') des tiges (2) lors du mouvement axial de la sonde par rapport au clou tubulaire et provoquant ensuite le mouvement vers la tête (1) du noeud (3), qui à son tour provoque une expansion radiale du clou tubulaire dans la zone proximale à ses tiges (2).

2. Un montage comprenant un support (6) et un clou intramédullaire conforme à la revendication 1, **caractérisé en ce que** le support (6) fonctionne avec la tête (1) du clou tubulaire, ce support étant le seul élément du montage fixé à l'os par des vis, précisément à l'extrémité proximale de celui-ci, et possédant un orifice axial gradué (8) pour la fixation de la tête (1) et une ailette radiale (9) comportant deux orifices (10) permettant de visser le support à l'os.

3. Un montage conforme à la revendication 2, **caractérisé en ce qu'**à l'intérieur de l'orifice axial (8) du support (6), précisément à l'extrémité extérieure de celui-ci, il y a une section filetée (12) pour la fixation d'un gabarit permettant le forage de l'os, qui est située dans l'alignement des orifices (10) du support (6), et pour l'implantation ultérieure d'un collier (13) capable de mouvoir la tige filetée (4) constituant la sonde afin de déplacer la protrusion (5) qu'elle porte vers la tête (1) du clou tubulaire.
